Europäisches Patentamt

European Patent Office

Office européen des brevets

(11) Veröffentlichungsnummer: **0 456 109 A2**

# (12) EUROPÄISCHE PATENTANMELDUNG

(21) Anmeldenummer: 91107077.9

(22) Anmeldetag: 02.05.91

(51) Int. Cl.⁵: **C11D 11/00**, C11D 3/395, C11D 3/22

(30) Priorität: 10.05.90 DE 4014978

(43) Veröffentlichungstag der Anmeldung:
13.11.91 Patentblatt 91/46

(84) Benannte Vertragsstaaten:
BE DE FR GB IT NL

(71) Anmelder: BASF Aktiengesellschaft
Carl-Bosch-Strasse 38
W-6700 Ludwigshafen(DE)

(72) Erfinder: Trieselt, Wolfgang, Dr.
Alwin-Mittasch-Platz 1
W-6700 Ludwigshafen(DE)
Erfinder: Dung, Bernhard, Dr.
Koenigsberger Strasse 4
W-6718 Gruenstadt(DE)
Erfinder: Mueller, Reinhard, Dr.
Q 7,23-26
W-6800 Mannheim(DE)
Erfinder: Oftring, Alfred, Dr.
Im Roehrich 49
W-6702 Bad Duerkheim(DE)
Erfinder: Perner, Johannes, Dr.
Ginsterweg 4
W-6730 Neustadt(DE)
Erfinder: Hotz, Johann
Landsberger Strasse 2
W-6703 Limburgerhof(DE)
Erfinder: Bohn, Michael, Dr.
Dammbruchstrasse 24
W-6700 Ludwigshafen(DE)

(54) Verfahren zur Herstellung einer körnigen Bleichaktivatorzusammensetzung.

(57) Herstellung einer körnigen Bleichaktivatorzusammensetzung, enthaltend
A) 40 bis 95 Gew.-% Pentaacetylglucose oder einer Mischung aus Pentaacetylglucose und weiteren üblichen Bleichaktivatoren und
B) 5 bis 60 Gew.-% eines oder mehrerer Zusatzstoffe aus den folgenden Stoffklassen
(1) basische anorganische Alkali- und Erdalkalimetallsalze,
(2) Mono-, Di- und Trialkylamine,
(3) chelatisierend wirkende organische Komplexbildner in Kombination mit Hydroxycarbonsäuren,
(4) wasserunlösliche anorganische Silicate,
(5) anionische Tenside,
(6) nichtionische Tenside,
(7) carboxylgruppenhaltige Polymerisate und
(8) Polysaccharide,
indem man
(a) Glucose mit Essigsäureanhydrid zu Pentaacetylglucose umsetzt,
(b) die hierbei entstehende Essigsäure bis auf einen Restgehalt von 0,2 bis 10 Gew.-% destillativ entfernt,
(c) das Reaktionsgemisch mit einem oder mehreren der Zusatzstoffe B und gegebenenfalls weiteren üblichen Bleichaktivatoren versetzt und
(d) das Produkt durch Konfektionierung in eine körnige Form überführt.

Die vorliegende Erfindung betrifft ein verbessertes Verfahren zur Herstellung einer körnigen Bleichaktivatorzusammensetzung, welche

A) 40 bis 95 Gew.-% Pentaacetylglucose oder einer Mischung aus Pentaacetylglucose und weiteren üblichen Bleichaktivatoren und

B) 5 bis 60 Gew.-% eines oder mehrerer Zusatzstoffe aus den folgenden Stoffklassen

(1) basische anorganische Alkali- und Erdalkalimetallsalze,

(2) Mono-, Di- und Trialkylamine,

(3) chelatisierend wirkende organische Komplexbildner in Kombination mit Hydroxycarbonsäuren,

(4) wasserunlösliche anorganische Silicate,

(5) anionische Tenside,

(6) nichtionische Tenside,

(7) carboxylgruppenhaltige Polymerisate und

(8) Polysaccharide

enthält.

Die körnigen Bleichaktivatorzusammensetzungen sind zum Teil neue Stoffe. Deshalb betrifft die Erfindung weiterhin diese neuen Stoffe.

Weiterhin betrifft die Erfindung wasch- und Reinigungsmittel, die die Bleichaktivatorzusammensetzung aus den Komponenten A und B (1) bis (8) enthalten.

Die EP-B 240 057 (1) betrifft eine körnige, keinen Phosphor enthaltende Bleichaktivatorzusammensetzung aus einem fein zerteilten Bleichaktivator, einem inerten, nicht alkalischen, wasserlöslichen anorganischen oder organischen Salz, einem wasserlöslichen, filmbildenden polymeren Material und einem Tonmaterial.

Die EP-A 156 977 (2) betrifft ein Herstellungsverfahren für einen granulierten Bleichaktivator aus einer aktiven acetylierten Verbindung, beispielsweise Pentaacetylglucose, und Natriumtripolyphosphat, wobei die aktive Verbindung mit einem Feuchtigkeitsgehalt von 20 bis 65 Gew.-% mit Natriumtripolyphosphat gemischt, anschließend granuliert und zum Schluß getrocknet wird.

In der EP-S 028 432 (3) wird als Bestandteil von Waschmittelzusammensetzungen ein teilchenförmiges Gemisch in Form von Körnern eines organischen Peroxysäurebleichprecursors, beispielsweise Glucosepentaacetat, mit Kieselsäure oder einem wasserunlöslichen Silicat und einem alkoxylierten nichtionischen oberflächenaktiven Mittel beschrieben.

Die USA 4 483 778 (4) betrifft eine körnige Peroxybleichaktivatorzusammensetzung, welche als Bindemittel oder einhüllendes Material nichtionische Tenside, Polyethylenglykole, Fettsäuren, anionische Tenside oder filmbildende Polymere wie Homo- und Copolymerisate von Acrylsäure, Hydroxyacrylsäure und Methacrylsäure oder Cellulosederivate enthält.

Für die übliche Verfahrensweise des Einarbeitens von Zusatzstoffen in die Bleichaktivatorsubstanz, wie beispielsweise in (2) beschrieben, ist es notwendig, die von der meist mit Essigsäureanhydrid durchgeführten Acetylierung herrührende Essigsäure vorher zu entfernen, indem man beispielsweise das acetylierte Produkt durch Wasser oder eine Wasser-Alkohol-Mischung ausfällt, abfiltriert und gegebenenfalls trocknet. Das ist aber eine umständliche und unökonomische Arbeitsweise, insbesondere im großtechnischen Maßstab. Die als Voraussetzung für eine Konfektionierung notwendige Kristallisation direkt aus der essigsäurehaltigen Reaktionsmischung mißlingt in der Regel. Geringe Restmengen an Essigsäure, die sich destillativ nicht mehr entfernen lassen, und weitere Nebenprodukte wirken hier störend.

Somit lag der vorliegenden Erfindung die Aufgabe zugrunde, eine konfektionierte Bleichaktivatorzusammensetzung bereitzustellen, welche direkt aus dem essigsäurehaltigen Rohprodukt der Acetylierungsstufe erhältlich ist.

Demgemäß wurde ein Herstellungsverfahren für die eingangs definierte körnige Bleichaktivatorzusammensetzung gefunden, welches dadurch gekennzeichnet ist, daß man

(a) Glucose mit Essigsäureanhydrid zu Pentaacetylglucose umsetzt,

(b) die hierbei entstehende Essigsäure bis auf einen Restgehalt von 0,2 bis 10 Gew.-% destillativ entfernt,

(c) das Reaktionsgemisch mit einem oder mehreren der Zusatzstoffe B und gegebenenfalls weiteren üblichen Bleichaktivatoren versetzt und

(d) das Produkt durch Konfektionierung in eine körnige Form überführt.

Glucose wird nach den üblichen Methoden mit der entsprechenden Menge an Essigsäureanhydrid zur rohen Pentaacetylglucose gemäß (a) umgesetzt.

Die Destillation (b) der Essigsäure erfolgt zweckmäßigerweise unter milden Bedingungen im Vakuum bei einem Druck von etwa 10 bis etwa 200 mbar, um die acetylierte Glucose keinen Zersetzungsprozessen auszusetzen. Ein Restgehalt von 0,5 insbesondere 0,2 Gew.-% Essigsäure kann hierbei nur schwer

2

unterschritten werden. Für die Fortführung des erfindungsgemäßen Herstellungsverfahrens ist ein Restgehalt an Essigsäure bis zu 10 insbesondere 5 Gew.-% noch tolerierbar.

Dem essigsäure- und nebenprodukthaltigen Reaktionsgemisch werden gemäß (c) die Zusatzstoffe B und gegebenenfalls weitere übliche Bleichaktivatoren zugemischt. Dabei ist es wegen der besseren Durchmischung vorteilhaft, wenn das Reaktionsgemisch noch in flüssigem oder zumindest plastisch verformbarem Zustand ist. Die Zugabe dieser Substanzen kann in Rühr- oder Knetapparaturen erfolgen. Bevorzugt wird jedoch die Verwendung eines oder mehrerer üblicher Extruder, weil die Durchmischung hierbei am schnellsten und am intensivsten erfolgt und außerdem direkt anschließend der Konfektionierungsschritt (d) vorgenommen werden kann. Außer der Extrusion können aber auch andere Konfektionierungsmethoden wie beispielsweise die Sprühkristallisation oder die Kompaktierung zum Einsatz gelangen. Den Extrudern können noch Kühl- und Zerkleinerungsvorrichtungen nachgeschaltet sein.

Besonders bevorzugt werden gleichsinnig laufende kontinuierliche Zweiwellen-Extruder zur Einarbeitung der Zusatzstoffe B und gegebenenfalls weiterer üblicher Bleichaktivatoren verwendet. Sie weisen vorteilhafterweise ein abreinigendes Profil auf. Das über Düsen auf eine bestimmte Zähigkeit kristallierte Produkt wird zweckmäßigerweise zu Endlosfäden ausgeformt und nach dem Abkühlen auf einem Kühlband zerkleinert. Die Kühlung des noch warmen flüssigen Rohproduktes aus (b) im Extruder erfolgt vorzugsweise mit Kühlwasser von 30 bis 70° C, damit die Zähigkeit der Schmelze nicht zu hoch ist und somit eine gute Verteilung der Zusatzstoffe B und gegebenenfalls weiterer üblicher Bleichaktivatoren nach dem Zumischen ermöglicht wird. Andererseits muß die Kühlung ausreichen, um genügend Kristallisationskeime zu erzeugen und eine Mindestzähigkeit zu erreichen, die erst eine Extrusion von formstabilen Fäden erlaubt.

Die Körner der Zusammensetzung haben eine Größe von etwa 0,5 bis etwa 3 mm. Je nach angewandter Konfektionierungsmethode haben die Körner Prill-, Granulat-, Pellet- oder Kompaktatform. Die Körner sind in der Regel weiß bis cremefarben und lösen sich in lauwarmem Wasser innerhalb kurzer Zeit leicht auf.

Zur Verbesserung der Bleichaktivatorwirkung kann die Komponente A neben Pentaacetylglucose noch weitere übliche Bleichaktivatoren enthalten. Das Gewichtsverhältnis der zusätzlichen Bleichaktivatoren zur Pentaacetylglucose beträgt hierbei üblicherweise 1:99 bis 90:10, vorzugsweise 1:99 bis 50:50.

Als zusätzliche Bleichaktivatoren kommen vor allem in Betracht:
- Acyloxybenzolsulfonsäuren und deren Alkali- und Erdalkalimetallsalze, z.B. Natrium-p-isononanoyloxy-benzolsulfonat oder Natrium-p-benzoyloxy-benzolsulfonat;
- N-diacylierte und N,N'-tetraacylierte Amine, z.B. N,N,N',N'-Tetraacetyl-methylendiamin und -ethylendiamin, N,N-Diacetylanilin, N,N-Diacetyl-p-toluidin oder 1,3-diacylierte Hydantoine wie 1,3-Diacetyl-5,5-dimethylhydantoin;
- Benz-(4H)1,3-oxazin-4-one, z.B. 2-Phenyl-benz-(4H)1,3-oxazin-4-on oder 2-Methyl-benz-(4H)1,3-oxazin-4-on;

Daneben kommen aber auch noch als zusätzliche Bleichaktivatoren in Frage:
- N-Alkyl-N-sulfonyl-carbonamide, z.B. N-Methyl-N-mesyl-acetamid oder N-Methyl-N-mesyl-benzamid;
- N-acylierte cyclische Hydrazide, acylierte Triazole oder Urazole, z.B. Monoacetyl-maleinsäurehydrazid;
- O,N,N-trisubstituierte Hydroxylamine, z.B. O-Benzoyl-N,N-succinyl-hydroxylamin, O-Acetyl-N,N-succinyl-hydroxylamin oder O,N,N-Triacetyl-hydroxylamin;
- N,N'-Diacyl-sulfurylamide, z.B. N,N'-Dimethyl-N,N'-diacetyl-sulfurylamid oder N,N'-Diethyl-N,N'-dipropionyl-sulfurylamid;
- Triacylcyanurate, z.B. Triacetylcyanurat oder Tribenzoylcyanurat;
- Carbonsäureanhydride, z.B. Benzoesäureanhydrid, m-Chlorbenzoesäureanhydrid oder Phthalsäureanhydrid;
- 1,3-Diacyl-4,5-diacyloxy-imidazoline, z.B. 1,3-Diacetyl-4,5-diacetoxy-imidazolin;
- Tetraacetylglycoluril und Tetrapropionylglycoluril;
- diacylierte 2,5-Diketopiperazine, z.B. 1,4-Diacetyl-2,5-diketopiperazin
- Acylierungsprodukte von Propylendiharnstoff und 2,2-Dimethylpropylendiharnstoff, z.B. Tetraacetylpropylendiharnstoff;
- α-Acyloxy-polyacyl-malonamide, z.B. α-Acetoxy-N,N'-diacetylmalonamid;
- Diacyl-dioxohexahydro-1,3,5-triazine, z.B. 1,5-Diacetyl-2,4-dioxohexahydro-1,3,5-triazin.

Eine bevorzugte körnige Bleichaktivatorzusammensetzung besteht aus

A) 45 bis 85 Gew.-% Pentaacetylglucose oder einer Mischung aus Pentaacetylglucose und weiteren üblichen Bleichaktivatoren,

B) 10 bis 50 Gew.-% eines oder mehrerer Zusatzstoffe B und

C) 0,3 bis 20 Gew.-% hierbei üblicher Hilfsstoffe und durch die Herstellung der Zusammensetzung bedingter Nebenprodukte.

Als basische anorganische Alkali- und Erdalkalimetallsalze (1) dienen vor allem Hydroxide wie Natriumhydroxid, Kaliumhydroxid, Magnesiumhydroxid oder Calciumhydroxid, Carbonate wie Natriumcarbonat, Natriumhydrogencarbonat, Kaliumcarbonat, Kaliumhydrogencarbonat, Magnesiumcarbonat, Magnesiumhydroxidhydrogencarbonat oder Calciumcarbonat sowie sonstige basisch reagierende Alkali- und Erdalkalimetallsalze schwacher Säuren wie wasserlösliches Natriumsilicat.

Als Mono-, Di und Trialkylamine (2) können am vorteilhaftesten solche mit mindestens einer längeren, etwa aus 8 bis 25 C-Atomen bestehenden Alkylgruppe eingesetzt werden. Als Beispiele sind hierfür N-Dodecylamin, N-Methyl-N-dodecylamin, N,N-Dimethyl-N-dodecylamin, N,N-Dimethyl-N-myristylamin, N,N-Dimethyl-N-cetylamin oder N,N-Dimethyl-N-stearylamin zu nennen.

Als chelatisierend wirkende organische Komplexbildner (3), welche in Kombination mit Hydroxycarbonsäuren wie Zitronensäure zum Einsatz gelangen, dienen vor allem Nitrilotriessigsäure, Ethylendiamintetraessigsäure, Isoserindiessigsäure und organische Phosphonsäuren wie Nitrilotrimethylenphosphonsäure, Ethylendiamintetramethylenphosphonsäure und Hydroxyethandiphosphonsäure sowie die zugehörigen Alkalimetallsalze.

Als wasserunlösliche anorganische Silicate (4) können Alkali- und Erdalkalimetall-Alumosilicate wie Zeolithe, beispielsweise Zeolith A, Aluminium-Magnesium-silicate (Bleicherden) wie Bentonit oder Attapulgit und Magnesiumsilicate wie Serpentin oder Talkum verwendet werden.

Als anionische Tenside (5) kommen z.B. Alkalimetallsalze von Fettsäuren, Alkylbenzolsulfonate, Alkansulfonate, α-Olefinsulfonate, Hydroxyalkansulfonate, α-Sulfofettsäureester, Alkylsulfate, Alkylethersulfate oder Fettalkoholethersulfate in Betracht.

Als nichtionische Tenside (6) dienen beispielsweise primäre oder sekundäre Alkoholoxalkylate wie Fettalkoholethoxylate sowie Alkylphenoloxalkylate, Fettsäurealkylolamide und Aminoxide.

Als carboxylgruppenhaltige Polymerisate (7) kommen vor allem Homopolymerisate der Acrylsäure und Methacrylsäure sowie Acrylsäure und/oder Methacrylsäure enthaltende Copolymerisate, z.B. Acrylsäure-Maleinsäure-Copolymerisate, sowie die zugehörigen Alkalimetall- oder Ammoniumsalze in Betracht.

Als Polysaccharide (8) dienen am vorteilhaftesten Stärke, Amylose sowie Derivate dieser natürlich vorkommenden Polysaccharide wie Carboxymethylcellulose oder sulfatierte Celluloseether.

Bevorzugt wird als Komponente B ein Alkalimetallsalz einer Fettsäure oder ein Gemisch solcher Salze oder ein Gemisch eines solchen Salzes mit anderen Zusatzstoffen B wie wasserunlöslichen anorganischen Silicaten, carboxylgruppenhaltigen Polymerisaten oder Polysacchariden eingesetzt. Unter Fettsäuren sind gesättigte oder ungesättigte aliphatische Monocarbonsäuren mit etwa 6 bis etwa 20, insbesondere 12 bis 18 C-Atomen, zu verstehen.

Beispiele für derartige Fettsäuresalze sind Natrium- und Kaliumstearat, Natrium- und Kaliumpalmitat, Natrium- und Kaliummyristat, Natrium- und Kaliumlaurat, Natrium- und Kaliumoleat sowie Natrium- und Kaliumlinolat. Meist werden diese Fettsäuresalze als Gemische in Form von Seifen eingesetzt. Übliche technische Seifen sind z.B. Kokosfettseiten und Talgfettseife.

Die körnigen Bleichaktivatorzusammensetzungen können noch übliche Hilfsstoffe in den hierfür üblichen Mengen enthalten. Übliche Hilfsstoffe, die z.B. als Sprengmittel, Dispergiermittel oder Konfektionierhilfsmittel dienen, sind beispielsweise anorganische Salze wie Natriumsulfat oder Natriumtriphoshat.

Als ein durch die Herstellung der Zubereitung bedingtes Nebenprodukt tritt u.a. ein Essigsäuresatz, beispielsweise ein Alkalimetallacetat wie Natrium- oder Kaliumacetat auf. Weiterhin können noch nicht acetylierte Glucose, unvollständig acetylierte Glucose und sonstige Glucose-Nebenprodukte in der Bleichaktivatorzusammensetzung vorhanden sein. Die Menge dieser Nebenprodukte kann bis zu etwa 10, in ungünstigen Fällen bis zu etwa 15 Gew.-% betragen. Die Nebenprodukte beeinträchtigen nicht die Bleichaktivator- und die Waschwirkung.

Gegenstand der vorliegenden Erfindung sind auch neue körnige Bleichaktivatorzusammensetzungen, welche

A) 40 bis 95 Gew.-% Pentaacetylglucose oder einer Mischung aus Pentaacetylglucose und weiteren üblichen Bleichaktivatoren und

B) 5 bis 60 Gew.-% eines oder mehrerer Zusatzstoffe aus den folgenden Stoffklassen

(1) basische anorganische Alkali- und Erdalkalimetallsalze,

(2) Mono-, Di- und Trialkylamine und

(3) chelatisierend wirkende organische Komplexbildner in Kombination mit Hydroxycarbonsäuren

enthalten.

Weiterhin sind Gegenstand der vorliegenden Erfindung Wasch- und Reinigungsmittel, die 0,5 bis 20 Gew.-%, vorzugsweise 1 bis 10 Gew.-%, der körnigen Bleichaktivatorzusammensetzung und die üblichen Bleichmittel in den üblichen Mengen enthalten. Insbesondere handelt es sich hierbei um Textilwaschmittel in Pulverform, denen die erfindungsgemäßen Mittel während oder im Anschluß an ihre Herstellung beigemischt werden. Übliche Bestandteile und Zusammensetzungen von Wasch- und Reinigungsmitteln sind dem Fachmann bekannt und brauchen deshalb hier nicht näher erörtert zu werden.

Die beschriebenen körnigen Bleichaktivatorzusammensetzungen lassen sich deshalb so gut herstellen, weil durch die Zugabe der Zusatzstoffe B zum essigsäure- und nebenprodukthaltigen Rohprodukt der Acetylierung von Glucose unerwartet eine rasche Verfestigung und Kristallisation zu einem Produkt ohne Klebeneigung erfolgt, wodurch die Konfektionierung erst ermöglicht wird. Ohne den Zusatz der Stoffe B bleibt der Effekt einer schnellen und reproduzierbaren Kristallisation weitgehend aus.

Der Zusatz der Stoffe B bewirkt außerdem die vollständige Bindung von störendem Essigsäuregeruch, erleichtert die Durchmischungs- und insbesondere die Extrusionsfähigkeit des Produktes und erhöht die Lagerstabilität der erfindungsgemäßen Bleichaktivatorzusammensetzung in alkalischen Wasch- und Reinigungsmitteln im Vergleich zu konfektionierten Bleichaktivatorzusammensetzungen des Standes der Technik.

Beispiele Nr. 1 bis 10

Zu 561 g (5,5 mol) Essigsäureanhydrid (Gehalt 91 Gew.-%) wurden bei Raumtemperatur unter Rühren 0,3 g 96 gew.-%ige Schwefelsäure geben. In die auf 60°C erwärmte Mischung wurden portionsweise 180 g (1,0 mol) wasserfreie Glucose eingetragen, wobei die Temperatur zwischen 60 bis 70°C gehalten wurde. Danach wurde 2 Stunden bei 60°C nachgerührt, die Schwefelsäure durch Zugabe von 0,5 g 50 gew.-%iger wäßriger Natronlauge neutralisiert und die entstandene Essigsäure im Vakuum bei 20 mbar bis auf einen Restgehalt von 4 Gew.-% abdestiliert. Man erhielt ca. 400 g Rohprodukt.

Dem noch warmen flüssigen Rohprodukt wurden in einem gleichsinnig laufenden Zweiwellen-Extruder der Baureihe ZDS-W der Firma Werner & Pfleiderer die in der folgenden Tabelle der hergestellten Bleichaktivatorzusammensetzungen aufgeführten Zusatz- und Hilfsstoffe beigemischt. Die Temperatur von Gehäuse und Schnecken wurde hierbei durch Kühlung auf ca. 50°C eingestellt und der Druck im Extruder betrug maximal 20 bar. Die mittlere Verweilzeit im Extruder lag bei ca. 1 min. Die extrudierten Fäden hatten einen Durchmesser von 1,5 bis 2 mm. Ihre Temperatur betrug nach Austritt aus den Düsen 60 bis 70°C. Sie wurden auf einem Kühlband auf Raumtemperatur abgekühlt und zu Granulaten mit einem Verhältnis von Länge zu Durchmesser von 1:1 bis 3:1 zerkleinert.

Die erhaltenen körnigen Bleichaktivatorzusammensetzungen behielten nach Lagerung in üblichen alkalischen Pulverwaschmitteln bei 38°C und einer Luftfeuchte von 76 % auch nach mehreren Wochen noch nahezu ihre ursprüngliche Aktivität.

5

Tabelle

Bleichaktivatorzusammensetzungen [Gew.-%] Nr. 1 bis 10

| Komponente | Beispiel Nr. 1 | 2 | 3 | 4 | 5 | 6 | 7 | 8 | 9 | 10 |
|---|---|---|---|---|---|---|---|---|---|---|
| Pentaacetylglucose | 46 | 52 | 78 | 83 | 66 | 60 | 56 | 45 | 50 | 66 |
| Natriumstearat | 46 | 32 | - | - | - | - | - | - | - | - |
| handelsübliche Kokosfettseife | - | - | 15 | 10 | - | - | - | - | - | 17 |
| handelsübliche Talgfettseife | - | - | - | - | 16 | 23 | 31 | 41 | 22 | - |
| Acrylsäure-Maleinsäure-Copolymer, Natriumsalz (Gew.-Verh. 70:30, mittl. Molmasse 70 000) | - | - | - | - | 4 | - | - | - | - | - |
| Polyacrylsäure (mittl. Molmasse 4500) | - | - | - | - | - | 4 | - | - | - | - |
| Zeolith A | - | - | - | - | - | - | 6 | - | - | - |
| Bentonit | - | - | - | - | - | - | - | 8 | - | - |
| Maisstärke | - | - | - | - | - | - | - | - | 10 | - |
| Carboxymethylcellulose | - | - | - | - | - | - | - | - | - | 5 |
| Natriumacetat | 5 | 3 | 4 | 4 | 5 | 4 | 4 | 3 | 5 | 4 |
| Glucose und Glucose-Nebenprodukte | 3 | 3 | 3 | 3 | 4 | 4 | 3 | 3 | 3 | 3 |
| Natriumsulfat | - | - | - | - | 5 | 5 | - | - | 10 | 5 |
| Natriumtriphosphat | - | 10 | - | - | - | - | - | - | - | - |

**Patentansprüche**

1. Verfahren zur Herstellung einer körnigen Bleichaktivatorzusammensetzung, enthaltend

A) 40 bis 95 Gew.-% Pentaacetylglucose oder einer Mischung aus Pentaacetylglucose und weiteren üblichen Bleichaktivatoren und

B) 5 bis 60 Gew.-% eines oder mehrerer Zusatzstoffe aus den folgenden Stoffklassen

(1) basische anorganische Alkali- und Erdalkalimetallsalze,

(2) Mono-, Di- und Trialkylamine,

(3) chelatisierend wirkende organische Komplexbildner in Kombination mit Hydroxycarbonsäuren,

(4) wasserunlösliche anorganische Silicate,

(5) anionische Tenside,

(6) nichtionische Tenside,

(7) carboxylgruppenhaltige Polymerisate und

(8) Polysaccharide,

dadurch gekennzeichnet, daß man

(a) Glucose mit Essigsäureanhydrid zu Pentaacetylglucose umsetzt,

(b) die hierbei entstehende Essigsäure bis auf einen Restgehalt von 0,2 bis 10 Gew.-% destillativ entfernt,

(c) das Reaktionsgemisch mit einem oder mehreren der Zusatzstoffe B und gegebenenfalls weiteren üblichen Bleichaktivatoren versetzt und

(d) das Produkt durch Konfektionierung in eine körnige Form überführt.

2. Verfahren zur Herstellung der körnigen Bleichaktivatorzusammensetzung nach Anspruch 1, dadurch gekennzeichnet, daß die körnige Bleichaktivatorzusammensetzung als Komponente A allein Pentaacetylglucose enthält.

3. Verfahren zur Herstellung der körnigen Bleichaktivatorzusammensetzung nach Anspruch 1 oder 2, dadurch gekennzeichnet, daß man die Konfektionierung (d) durch Extrusion vornimmt.

4. Körnige Bleichaktivatorzusammensetzung, enthaltend

A) 40 bis 95 Gew.-% Pentaacetylglucose oder einer Mischung aus Pentaacetylglucose und weiteren üblichen Bleichaktivatoren und

B) 5 bis 60 Gew.-% eines oder mehrerer Zusatzstoffe aus den folgenden Stoffklassen

(1) basische anorganische Alkali- und Erdalkalimetallsalze,

(2) Mono-, Di- und Trialkylamine,

(3) chelatisierend wirkende organische Komplexbildner in Kombination mit Hydroxycarbonsäuren.

5. Wasch- und Reinigungsmittel enthaltend 0,5 bis 20 Gew.-% der gemäß den Ansprüchen 1 bis 3 hergestellten körnigen Bleichaktivatorzusammensetzung.

6. Wasch- und Reinigungsmittel enthaltend 0,5 bis 20 Gew.-% der körnigen Bleichaktivatorzusammensetzung gemäß Anspruch 4.